# FASCICULE DE BREVET EUROPEEN

(11) **EP 0 588 860 B1**
(45) Date de publication et mention de la délivrance du brevet: **06.12.1995**
(21) Numéro de dépôt: 92911679.6
(22) Date de dépôt: 10.06.1992
(51) Int. Cl.: A61F 13/15

(54) **ARTICLE D'HYGIENE ABSORBANT JETABLE AVEC POCHES DE CEINTURE AMELIORANT L'ETANCHEITE**
DEHNBARER ABSORBIERENDER GEGENSTAND
DISPOSABLE ABSORBING SANITARY ARTICLE WITH WAIST POCKETS IMPROVING THE FLUID-TIGHTNESS

(30) Priorité: 12.06.1991 FR 9107164
(43) Date de publication de la demande: 30.03.1994
(73) Titulaire: PEAUDOUCE, 59126 Linselles (FR)
(72) Inventeur: VANDEMOORTELE, Philippe, F-59000 Lille (FR); LEROY, André, F-59420 Mouvaux (FR)
(74) Mandataire: Casalonga, Axel
(86) Numéro de dépôt international: FR9200525
(87) Numéro de publication internationale: WO9222271

(56) Documents cités:
- EP-A- 0 109 126
- EP-A- 0 203 712
- EP-A- 0 264 238
- EP-A- 0 376 022

## Description

La présente invention se rapporte à des articles d'hygiène absorbants jetables, notamment des couches-culottes, du type comprenant une feuille de support extérieure imperméable aux liquides, une feuille de couverture intérieure, un coussin absorbant disposé entre lesdites feuilles, des éléments élastiques longitudinaux fixés à l'état tendu à ladite feuille de support, à l'extérieur des bords longitudinaux du coussin absorbant, et des moyens d'attache pour fermer l'article d'hygiène autour de la taille d'un utilisateur.

Pour améliorer l'étanchéité en général de ces articles d'hygiène dans la zone d'entre-jambes, ainsi que l'effet de confinement de l'urine et des matières fécales, et cela même dans le cas de défécations diarrhétiques brutales que l'on rencontre fréquemment dans le cas d'enfants en bas âge, il a déjà été proposé, par exemple par la demande de brevet GB-A-2 161 059, de prévoir en plus, sur la face intérieure de la feuille de couverture, deux rabats ou volets latéraux espacés transversalement s'étendant sensiblement le long des bords longitudinaux de l'article d'hygiène. Ces volets présentent chacun une partie proximale reliée à la feuille de couverture et une partie distale comportant des éléments élastiques longitudinaux tendus.

Du fait de leurs éléments élastiques tendus, ces volets latéraux se trouvent en permanence plaqués contre la peau de l'utilisateur, quels que soient les mouvements effectués par ce dernier, ce qui n'est pas toujours le cas pour les éléments élastiques longitudinaux fixés à la feuille de support extérieure, notamment du fait de la présence du coussin absorbant et de la rigidité et de l'épaisseur du coussin. Toutefois, cette amélioration ne concerne que l'étanchéité transversale ou latérale (à l'entre-jambes) et non pas l'étanchéité longitudinale (à la ceinture).

Les tentatives d'amélioration de l'étanchéité à la ceinture par la prévision d'une ceinture élastifiée et/ou d'une bande transversale imperméable recouvrant le bord transversal du coussin absorbant n'ont pas donné entière satisfaction.

Par les demandes de brevet EP-A-0 264 238 et EP-A-0 376 022, il a déjà été proposé de prévoir, sur un article d'hygiène du type comportant intérieurement deux rabats ou volets latéraux élastiques, au moins au voisinage d'un bord transversal de l'article, un élément transversal formant poche de ceinture s'étendant entre lesdits volets en étant disposé, soit au-dessus soit en dessous desdits volets. Cet élément est fixé à l'état tendu transversalement aux parties sous-jacentes de l'article d'hygiène par son bord transversal correspondant au bord transversal de l'article d'hygiène, ainsi que sur toute ou partie de ses bords longitudinaux, tandis que son autre bord transversal reste libre, ce qui permet à l'élément de s'ouvrir à la manière d'une poche à l'endroit de ce bord transversal libre intérieur. Toutefois, ces éléments de poche ne donnent pas entière satisfaction dans la mesure où ils ne procurent pas une étanchéité suffisante, notamment vis-à-vis des migrations de liquide en direction de la ceinture. De plus, la fabrication d'un article d'hygiène comportant de tels éléments de poche est relativement compliquée et entraîne donc une augmentation sensible du coût de l'article.

Enfin, par le brevet US-A- 4 662 877, on connaît une couche-culotte dont la feuille de couverture est recouverte, sur toute sa longueur, d'une feuille supplémentaire rectangulaire perméable aux liquides, de largeur réduite, fixée à la feuille de couverture selon ses quatre côtes et pourvue d'une ouverture centrale de forme générale rectangulaire dont les bords longitudinaux opposés sont garnis d'éléments élastiques tendus. Ces éléments élastiques longitudinaux tendus soulèvent la feuille supplémentaire de la feuille de couverture et du coussin absorbant sous-jacent le long des bords longitudinaux de ladite ouverture, formant ainsi des sortes de barrières latérales. Toutefois, cet effet de soulèvement est limité, entre autres en raison de la longueur réduite de ces éléments élastiques. Il en est de même du soulèvement, donc de l'effet de poche de ceinture des bords transversaux de ladite ouverture sous la tension des éléments élastiques longitudinaux.

La présente invention vise à perfectionner les articles d'hygiène du type à rabats ou volets latéraux et à poche (s) de ceinture de manière à améliorer d'une manière générale l'effet d'étanchéité longitudinale, c'est-à-dire à l'endroit de la ceinture, notamment du point de vue de la migration des liquides. L'invention vise également à réaliser une couche-culotte de ce type qui, tout en étant d'une meilleure efficacité, peut être fabriquée à un coût réduit.

L'article d'hygiène absorbant jetable conforme à l'invention, notamment une couche-culotte, présente une forme générale sensiblement rectangulaire avec des bords longitudinaux opposés et des bords transversaux opposés. Cet article comprend, de l'extérieur vers l'intérieur, une feuille de support imperméable aux liquides, un coussin absorbant disposé sur la face intérieure de la feuille de support, et une feuille de couverture. Les dimensions du coussin absorbant sont inférieures à celles de la feuille de support et la feuille de couverture recouvre la face intérieure du coussin absorbant et de la feuille de support et est reliée à cette dernière sur le pourtour du coussin absorbant. L'article d'hygiène comprend, en outre, des éléments élastiques longitudinaux fixés à l'état tendu à la feuille de support, à l'extérieur des bords latéraux du coussin absorbant. De plus, l'article d'hygiène comprend deux volets latéraux espacés transversalement, disposés sur la face intérieure de la feuille de couverture, sensiblement le long des bords longitudinaux de l'article d'hygiène, lesdits volets présentant chacun une partie proximale reliée à la feuille de couverture et une partie distale comportant des éléments élastiques longitudinaux tendus. Par ailleurs, l'article d'hygiène comprend des moyens d'attache au voisinage de l'un de ses bords transversaux, pour fermer l'article d'hygiène autour de la taille d'un utilisateur de telle manière que l'article d'hygiène définisse une partie avant et une partie arrière correspondant respectivement aux deux zones d'extrémité proches desdits bords transversaux opposés de l'article d'hygiène, et une partie d'entre-jambes correspondant à la zone intermédiaire située entre lesdites zones d'extrémité. Selon l'invention, l'article d'hygiène comprend, en outre, en dessous desdits volets latéraux, une feuille supplémentaire perméable aux liquides s'étendant depuis l'un au moins desdits bords transversaux au moins par-dessus le bord transversal correspondant du coussin absorbant. Parmi les deux feuilles constituées par la feuille de couverture et ladite feuille supplémentaire, une première qui est la plus éloignée du coussin absorbant comporte, au-dessus du coussin absorbant, au voisinage dudit bord transversal du coussin absorbant, une découpe transversale s'étendant entre lesdites parties proximales des volets latéraux. Ladite première feuille est par ailleurs munie d'un élément élastique transversal tendu longeant ladite découpe sur le côté tourné vers le bord transversal correspondant de l'article d'hygiène. Ladite feuille est en outre fixée à la seconde desdites deux feuilles au moins le long dudit bord transversal de l'article d'hygiène et dans le prolongement des deux extrémités dudit élément élastique transversal, de manière que ladite première feuille constitue une poche d'étanchéité de ceinture ouverte à l'endroit de ladite découpe.

Ladite feuille supplémentaire peut être disposée entre le coussin absorbant et la feuille de couverture et constituer ladite seconde feuille.

Ladite feuille supplémentaire peut également être disposée sur la face intérieure de ladite feuille de couverture et constituer ainsi ladite première feuille.

Ladite feuille supplémentaire peut être soit continue, c'est-à-dire s'étendre dans le sens longitudinal de l'article d'hygiène d'un bord transversal à l'autre de cet article, ou bien être limitée à la partie avant et/ou la partie arrière de l'article d'hygiène.

La fixation de la première feuille à la seconde feuille peut se faire avantageusement par un collage en U le long du bord transversal de la première feuille correspondant au bord transversal de l'article d'hygiène et le long des bords longitudinaux de la première feuille, sur tout ou partie de la longueur de ces bords.

De préférence, cette fixation s'effectue par un collage en rectangle, suivant une ligne transversale longeant le bord transversal de l'article d'hygiène, suivant deux lignes longitudinales longeant les bords longitudinaux de la première feuille, et suivant une ligne transversale longeant la découpe transversale de la première feuille sur le côté éloigné du bord transversal correspondant de l'article d'hygiène.

La partie de la première feuille située entre ladite découpe transversale et le bord transversal correspondant de l'article d'hygiène est de préférence imperméable.

Suivant un mode de réalisation de l'invention, la feuille supplémentaire constituant ladite première feuille et s'étendant sur toute la longueur de l'article d'hygiène est fixée à la feuille de couverture, sur toute la longueur de l'article d'hygiène, par deux lignes de liaison longitudinales en retrait par rapport aux bords longitudinaux de la feuille supplémentaire, des éléments élastiques tendus étant fixés le long desdits bords longitudinaux de la feuille supplémentaire. Ainsi, les parties de ladite feuille supplémentaire situées entre lesdites lignes longitudinales de liaison et lesdits bords longitudinaux constituent les rabats ou volets améliorant l'étanchéité transversale.

Dans ce cas, la feuille supplémentaire perméable aux liquides peut être avantageusement enduite ou traitée imperméable ou hydrophobe dans la zone desdits volets.

En se référant aux dessins schématiques annexés, on va décrire ci-après plus en détail plusieurs modes de réalisation illustratifs et non limitatifs d'un article d'hygiène conforme à l'invention; sur les dessins :
la figure 1 est une vue sur la face intérieure d'une couche-culotte conforme à l'invention disposée à plat;
la figure 2 est une coupe partielle à plus grand échelle suivant II-II de la figure 1;
la figure 3 est une coupe partielle à plus grande échelle suivant III-III de la figure 1;
la figure 4 est une vue sur la face intérieure d'un autre mode de réalisation d'une couche-culotte conforme à l'invention, disposée à plat;
la figure 5 est une coupe partielle à plus grande échelle suivant V-V de la figure 4;
la figure 6 est une coupe partielle à plus grande échelle suivant VI-VI de la figure 4;
la figure 7 est une coupe correspondant à celle de la figure 6 d'une variante sur laquelle les volets latéraux et les poches de ceinture sont formés à partir d'une seule et même feuille perméable aux liquides.
la figure 8 est une vue sur la face intérieure d'encore un autre mode de réalisation d'une couche-culotte conforme à l'invention, disposée à plat;
les figures 9 et 10 sont des coupes à plus grande échelle suivant IX-IX et suivant X-X de la figure 8.

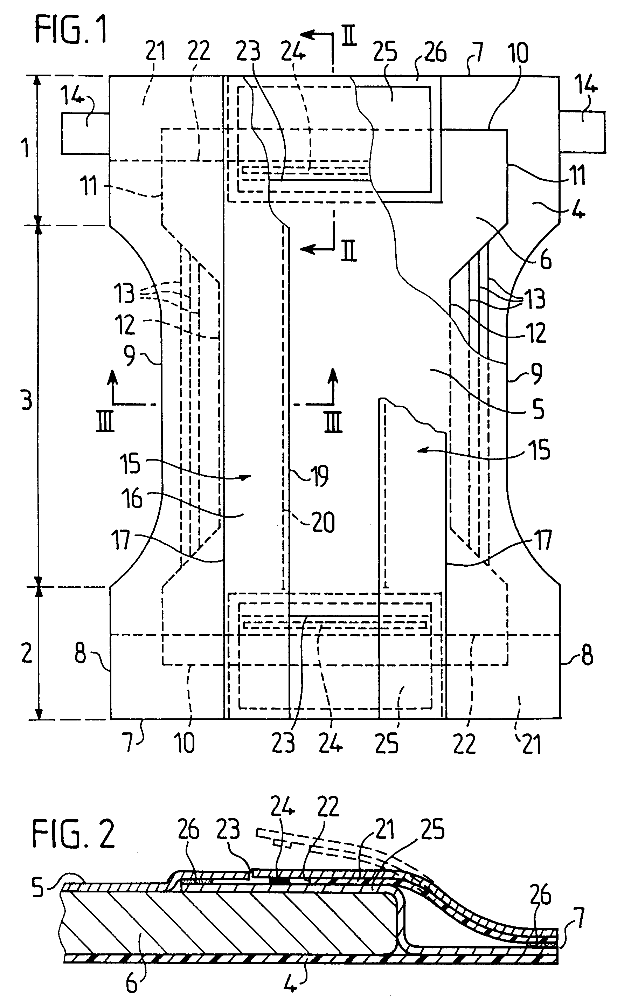

La couche-culotte à jeter telle qu'illustrée par les figures 1 à 3 est une couche-culotte dite anatomique ou en sablier, ayant une forme générale rectangulaire avec deux découpes latérales opposées permettant de définir, dans le sens de la longueur de la couche-culotte, une partie arrière 1 correspondant à une zone d'extrémité longitudinale, une partie avant 2 correspondant à l'autre zone d'extrémité longitudinale et une partie d'entre-jambes 3 correspondant à la zone intermédiaire de la couche-culotte. Dans la partie d'entre-jambes 3, la couche-culotte présente une largeur plus faible que dans la partie avant 1 et dans la partie arrière 2.

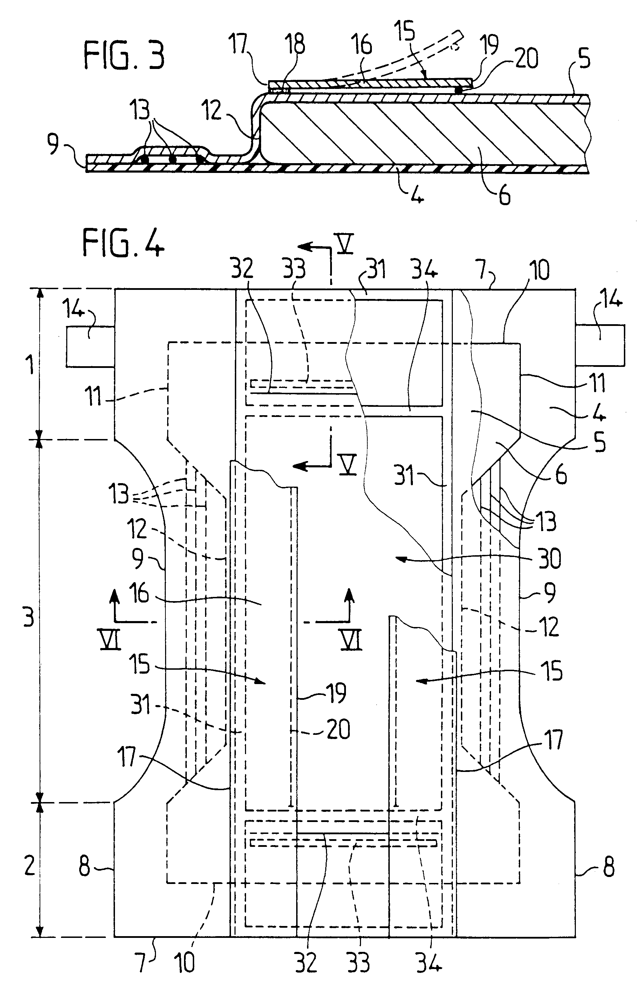

La structure générale de la couche-culotte suivant les figures 1 à 3, correspond à celle des couches-culottes classiques, comprenant de l'extérieur vers l'intérieur, c'est-à-dire de bas en haut sur les figures 2 et 3 une feuille de support extérieure 4 imperméable aux liquides, une feuille de couverture intérieure 5 perméable aux liquides, et un coussin absorbant 6 disposée entre les deux feuilles 4 et 5.

Selon la figure 1, les deux feuilles 4 et 5 ont la même taille et la même forme, à savoir une forme générale rectangulaire avec deux bords transversaux 7 rectilignes opposés et deux bords longitudinaux 8 opposés comportant chacun une échancrure 9 sensiblement au milieu de sa longueur, ce qui donne aux deux feuilles 4 et 5 leur forme en sablier, les échancrures 9 définissant la partie d'entre-jambes 3 de largeur réduite.

Le matelas absorbant 6 disposé entre les deux feuilles 4 et 5 qui est également en forme de sablier, présente une taille réduite par rapport à celle des feuilles 4 et 5 et est centré par rapport à ces feuilles 4 et 5 de manière que ses deux bords transversaux 10 rectilignes ainsi que ses deux bords longitudinaux 11 chacun muni d'une échancrure 12 se trouvent respectivement en retrait vers l'intérieur par rapport aux bords correspondants 7 et 8 des deux feuilles 4 et 5.

La couche-culotte comporte par ailleurs, de façon connue en soi, deux éléments élastiques 13 longitudinaux, constitués dans l'exemple représenté chacun des trois brins élastiques espacés, fixés à l'état tendu à la feuille extérieure 4, au moins dans la partie d'entre-jambes 3, entre le fond des échancrures 12 du coussin absorbant 6 et le fond des échancrures 9 des feuilles 4 et 5.

Deux attaches adhésives 14 sont prévues de façon connue en soi sur la partie arrière 1, en vue de la fermeture de la couche-culotte autour de la taille d'un utilisateur, les attaches 14 venant alors coopérer avec la partie avant 2 de la couche-culotte.

Il est à noter que les moyens de fixation de la feuille intérieure 5 à la feuille extérieure 4, autour du coussin absorbant 6, les moyens de fixation du coussin absorbant 6 sur la feuille 4 ainsi que les moyens de fixation des éléments élastiques 13 à la feuille extérieure 4 ne sont pas représentés, ces moyens connus en soi pouvant être constitués par exemple par des lignes ou enductions de colle.

La feuille intérieure 5 porte sur sa face intérieure, c'est-à-dire la face visible sur la figure 1, deux volets ou rabats latéraux 15 d'étanchéité ou de barrière. Tel que cela apparaît sur la figure 3, chaque rabat 15 est formé d'un ruban 16 d'un matériau en feuille, perméable ou imperméable aux liquides, s'étendant sur toute la longueur de la couche-culotte. Le ruban 16 est relié à son extrémité proximale, correspondant à son bord longitudinal extérieur 17, par une ligne de colle 18 à la feuille de couverture 5, à savoir dans l'exemple représenté dans une position légèrement en retrait vers l'intérieur par rapport au fond de l'échancrure 12 du matelas absorbant 6. A son extrémité distale 19 correspondant à son bord longitudinal intérieur, le ruban 16 porte un élément élastique 20, fixé de préférence par collage, à l'état tendu au ruban 16, sur la partie médiane de la longueur de ce dernier correspondant à la partie d'entre-jambes 3.

Il est à noter que les rubans 16 constituant les volets 15 sont également fixés sur toute leur largeur à la feuille de couverture 5 le long des deux bords transversaux 7.

Ces volets ou rabats latéraux 15 connus en soi sont destinés à améliorer l'effet de barrière et d'étanchéité dans le sens latéral, les éléments élastiques 20 tendus ayant pour fonction de soulever les volets 15 par rapport a la feuille de couverture 5, comme indiqué en tirets sur la figure 3, et de les appliquer contre la peau de l'utilisateur, de manière à leur donner une certaine indépendance par rapport aux parties restantes de la couche-culotte et en particulier par rapport au matelas absorbant 6, de sorte que les volets 15 puissent suivre les mouvements de l'utilisateur tout en restant plaqués contre la peau de ce dernier.

En outre, une bande transversale 21 imperméable aux liquides est prévue sur la feuille de couverture 5, dans la zone de chaque bord transversal 7 de la couche-culotte. Dans l'exemple représenté, la bande 21 est constituée par une feuille imperméable doublant la feuille de couverture 5 sur la face tournée vers le coussin absorbant 6, cette bande 21 s'étendant sur toute la largeur de la feuille 5 et ayant, dans le sens de la longueur de la couche-culotte, une dimension telle que la bande 21 chevauche le coussin absorbant 6, son bord intérieur 22 se trouvant en retrait vers l'intérieur par rapport au bord transversal 10 correspondant du coussin 6.

Tel que cela apparaît sur les figures 1 et 2, la feuille de couverture 5 comporte, aussi bien dans la partie arrière 1 que dans la partie avant 2 de la couche-culotte, une découpe transversale 23 s'étendant en position médiane sur une longueur légèrement inférieure à la distance transversale séparant l'un de l'autre les bords extérieurs 17 des deux volets latéraux 15. Chacune des découpes 23 est décalée vers l'intérieur par rapport au bord intérieur 22 de la bande imperméable 21 de la feuille de couverture 5 de manière que la découpe 23 se trouve au-dessus du coussin 6.

Par ailleurs, la feuille de couverture 5 porte, sur sa face intérieure tournée vers le coussin absorbant 6, dans chacune des parties 1 et 2 de la couche-culotte, un élément élastique 24 transversal longeant ladite découpe 23, entre cette dernière et le bord intérieur 22 de la bande transversale imperméable 21, l'élément élastique 24 étant fixé à l'état tendu à la feuille 5.

Enfin, un tronçon 25 de feuille perméable aux liquides s'étend, dans chacune des deux parties 1 et 2 de la couche-culotte, au milieu de la largeur de cette dernière, en dessous de la feuille de couverture 5, depuis le bord transversal 7 correspondant de la couche-culotte vers l'intérieur, par-dessus le bord transversal 10 correspondant du matelas absorbant 6, jusqu'au-delà de la découpe transversale 23 correspondante de la feuille de couverture 5. Le tronçon de feuille 25 présente, dans le sens transversal de la couche-culotte, une dimension légèrement inférieure à la distance séparant l'un de l'autre les bords longitudinaux extérieurs 17 des deux volets latéraux 15.

Le tronçon de feuille 25 rectangulaire est fixé sur tout son pourtour, par un encollage rectangulaire 26, à la feuille de couverture 5.

Dans le mode de réalisation illustré par les figures 4 à 6, on retrouve une couche-culotte en sablier, avec une partie arrière 1, une partie avant 2 et une partie d'entre-jambes 3 de largeur réduite. Cette couche-culotte est également formée d'une feuille de support 4 extérieure, imperméable aux liquides, d'une feuille de couverture 5 intérieure, perméable aux liquides, d'un coussin absorbant 6 disposé entre les deux feuilles 4, 5 de manière que ses bords 10, 11, 12 se trouvent en retrait par rapport aux bords correspondants 7, 8, 9 des deux feuilles 4 et 5, d'éléments élastiques longitudinaux 13 disposés de part et d'autre du coussin absorbant 6 dans la zone d'entre-jambes, d'attaches adhésives 14 et de deux volets ou rabats latéraux 15 constitués par des rubans 16 fixés au voisinage de leur bord longitudinal extérieur 17, correspondant à leur extrémité proximale, par une ligne de colle 18 sur la feuille de couverture 5 et portant au voisinage de leur bord longitudinal intérieur 19, constituant leur extrémité distale, un élément élastique 20 longitudinal fixé à l'état tendu.

Dans ce mode de réalisation, un ruban 30 de feuille perméable aux liquides, ayant une largeur légèrement inférieure à la distance séparant, transversalement à la longueur de la couche-culotte, l'une de l'autre les deux lignes de colle 18 par lesquelles les volets latéraux 15 sont fixés sur la feuille de couverture 5, s'étend en position médiane sur toute la longueur de la couche-culotte, sur la face intérieure de la feuille de couverture 5. Le ruban 30 est fixé sur tout son pourtour par un encollage rectangulaire 31 sur la feuille de couverture 5 et comporte, dans la partie arrière 1 et dans la partie avant 2, au-dessus du coussin absorbant 6 chaque fois une découpe transversale 32 s'étendant transversalement à la longueur de la couche-culotte sur une longueur légèrement inférieure à la largeur du ruban 30.

Chaque découpe transversale 32 du ruban 30 est longée, sur le côté tourné vers le bord transversal 7 correspondant de la couche-culotte, par un élément élastique transversal 33 fixé à l'état tendu à la face du ruban 30 tourné vers le coussin absorbant 6 et, sur le côté opposé, par une bande transversale de colle 34 qui rejoint et complète l'encollage rectangulaire 31 faisant tout le pourtour du ruban 30.

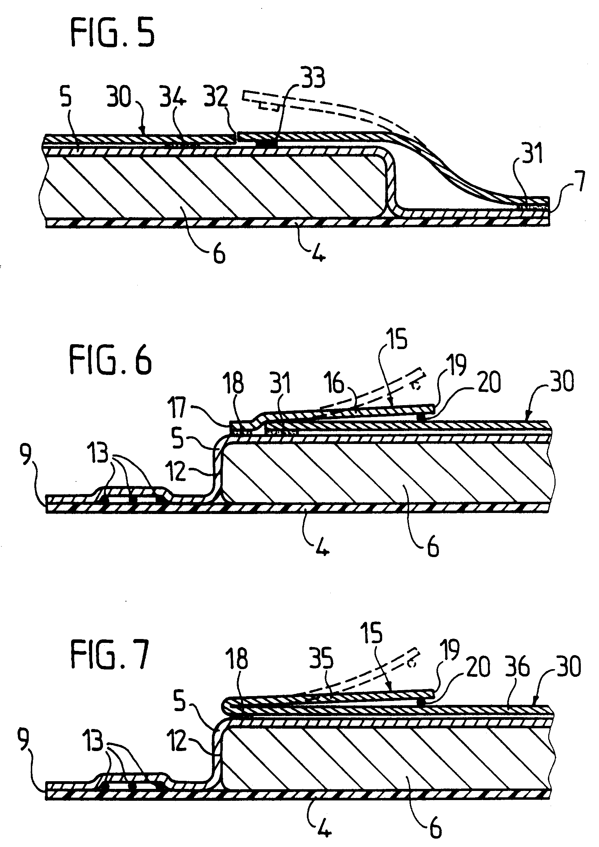

La variante suivant la figure 7 diffère du mode de réalisation suivant les figures 4 à 6 uniquement par le fait que les deux volets latéraux 15 sont réalisés d'une seule pièce avec le ruban de feuille 30 recouvrant la feuille de couverture 5 sur toute la longueur de la couche-culotte. A cet effet, le ruban de feuille 30 présente une largeur supérieure à la largeur du coussin absorbant 6 dans la partie d'entre-jambes 3 (distance transversale séparant les fonds des échancrures 12 du coussin 6), et les deux parties latérales 35 du ruban 30 situées à l'extérieur des lignes de colle longitudinales 18 par lesquelles la partie médiane 36 du ruban 30 est fixée à la feuille de couverture 5, au-dessus du coussin absorbant 6, sont repliées vers l'intérieur pour constituer les volets d'étanchéité latéraux 15 munis d'éléments élastiques longitudinaux 20 tendus à leur extrémité distale 19.

La partie restante de la couche-culotte correspond exactement au mode de réalisation des figures 4 à 6.

Par conséquent, dans le mode de réalisation de la figure 7, les volets latéraux 15 améliorant l'étanchéité à l'entre-jambes et les poches améliorant l'étanchéité à la ceinture sont constitués par un même élément, à savoir le ruban 30.

Dans les deux modes de réalisation suivant les figures 4 à 6 et suivant la figure 7, dans lesquels les découpes transversales 32 sont pratiquées dans un ruban 30 s'étendant, sur toute la longueur de la couche-culotte, par dessus la feuille de couverture 5, le ruban 30 constitué par un matériau perméable aux liquides, par exemple un non-tissé, peut être rendu imperméable ou hydrophobe dans la zone des poches de ceinture, par exemple par une enduction ou un autre traitement.

Une telle enduction ou un tel traitement imperméable ou hydrophobe pourrait également être prévu, dans le mode de réalisation de la figure 7, sur les parties 35 du ruban constituant les volets latéraux 15.

De façon analogue, les rubans 16 constituant les volets latéraux 15 dans les modes de réalisation suivant les figures 1 à 3 et 4 à 6 peuvent être avantageusement constitués par des rubans de non-tissé rendus hydrophobes ou imperméables.

Par ailleurs, dans le mode de réalisation des figures 1 à 3, la feuille de couverture 5 dans laquelle sont pratiquées les découpes transversales 22 pourrait être rendue imperméable ou hydrophobe, par enduction ou traitement, dans la zone des poches de ceinture, de préférence sur toute la largeur de la couche-culotte.

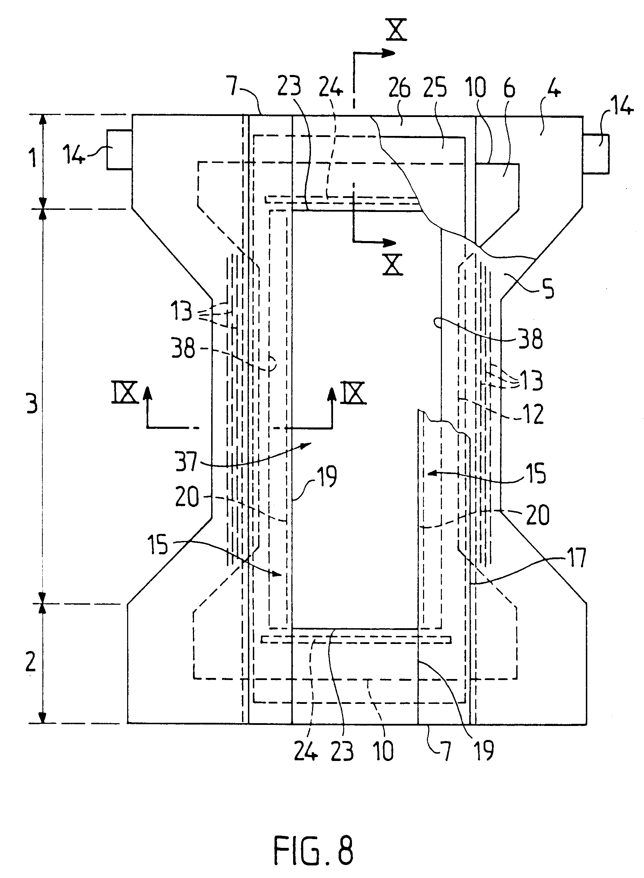

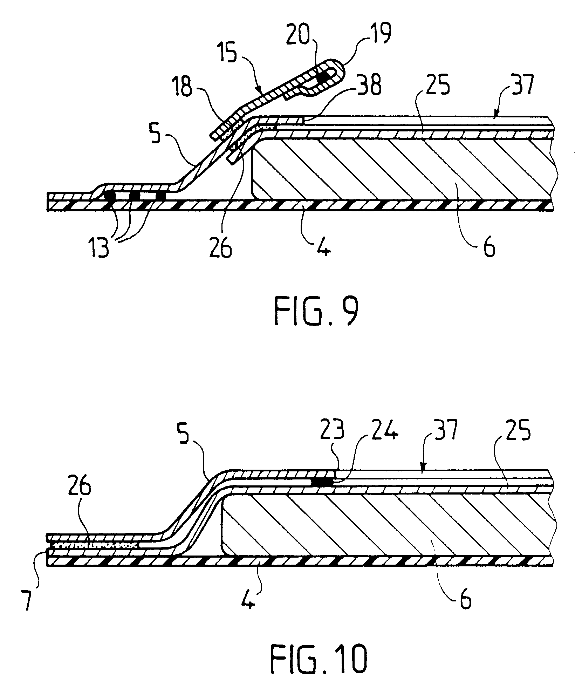

La couche-culotte du mode de réalisation suivant les figures 8 à 10 comprend une feuille de support 4 avec des éléments élastiques longitudinaux 13, un coussin absorbant 6, une feuille de couverture 5 recouvrant complètement la feuille de support 4 et le coussin 6, et deux volets latéraux 15. De plus, un ruban 25 ayant une largeur légèrement supérieure à la largeur du coussin absorbant 6 dans la partie d'entre-jambes 3 s'étend entre la feuille de couverture 5 et le coussin 6, sur toute la longueur de la couche-culotte, entre les deux bords transversaux 7 opposés de cette dernière. Ce ruban constitué par un matériau perméable aux liquides, par exemple un non-tissé, est fixé sur tout son pourtour, par exemple par une bande rectangulaire de colle 26, à la feuille de couverture 5.

La feuille de couverture 5, réalisée en un matériau souple tel qu'un non-tissé, hydrophobe ou de préférence imperméable aux liquides, comporte en position centrale une ouverture 37 de forme générale rectangulaire, allongée dans la direction longitudinale de la couche-culotte, de largeur inférieure à la largeur du coussin 6 dans la partie d'entre-jambes 3. Cette ouverture 37 est définie par deux découpes ou bords transversaux 23 qui sont nettement en retrait par rapport aux zones de collage 26 transversales et sont munis chacun d'un élément élastique 24 transversal tendu, et par deux découpes ou bords longitudinaux opposés 38 qui joignent les extrémités desdits bords 23 et le long desquels sont fixés, par exemple par des bandes de colle 18, les deux volets latéraux 15 qui s'étendent sur toute la longueur de la couche-culotte et portent chacun un élément élastique tendu 20 au voisinage de leur partie distale 19.

Ainsi, les deux bords transversaux élastifiés 23 de l'ouverture 37 de la feuille de couverture 5 définissent deux poches d'étanchéité de ceinture qui s'étendent entre lesdits bords 23 et les bandes de colle 26 et qui, en combinaison avec les deux volets latéraux 15, constituent une poche d'étanchéité empêchant les fuites de liquides ou de solides sur toute la périphérie de la couche-culotte.

Il y a lieu de noter que les découpes ou bords transversaux 23, au lieu d'être rectilignes, pourraient également être incurvés, notamment avec une convexité vers l'extérieur, ce qui donnerait à l'ouverture 37 une forme arrondie à ses deux extrémités longitudinales.

Par ailleurs, au lieu de réaliser l'ouverture 37 par deux découpes transversales et deux découpes longitudinales joignant les extrémités des découpes transversales, il serait possible de pratiquer dans la feuille de couverture une découpe en I, avec deux découpes transversales et une seule découpe longitudinale médiane, et de replier vers le dessus ou vers le dessous les deux rabats ainsi formés. Dans ce cas, les deux rabats peuvent être de préférence élastifiés au voisinage de leurs lignes de pliage. Il est alors possible de supprimer les volets latéraux 15, les rabats élastifiés étant à même d'assumer la fonction des volets, c'est-à-dire de créer des barrières latérales d'étanchéité relativement indépendantes du coussin absorbant sou-jacent.

Bien que dans tous les modes de réalisation représentés, les éléments élastiques 20 ne s'étendent que sur la partie médiane de la longueur des articles d'hygiène, correspondant à la partie d'entre-jambes 3, il peut être avantageux de faire en sorte que les éléments élastiques 20 s'étendent au moins jusqu'au - delà des découpes transversales 23, 32 et même jusqu'aux bords transversaux 7, ce qui améliore le soulèvement des poches de ceinture.

Bien que tous les exemples représentés illustrent des couches-culottes pour enfants ou pour incontinents adultes, l'invention est applicable également à d'autres articles d'hygiène ayant la même structure générale. Quelle que soit la nature de ces articles d'hygiène, ces derniers peuvent également comporter, dans le cadre de l'invention, une seule poche d'étanchéité de ceinture, soit à l'avant, soit à l'arrière.

En outre, l'invention est également applicable à des articles d'hygiène qui ne sont pas en forme de sablier, mais par exemple de forme rectangulaire.

Une autre possibilité applicable à tous les modes de réalisation représentés consiste à doubler le tronçon de ruban 25 ou le ruban 30 d'une feuille imperméable élastique dans la ou les zones devant être imperméables et d'utiliser l'élasticité de cette feuille, de préférence du type thermo-activable, pour remplacer l'élément élastique 24 ou 33 rapporté.

Les éléments élastiques 24, 33 rapportés le long des découpes transversales 23, 32 peuvent être constitués, dans le cadre de l'invention, par un ou plusieurs brins élastiques, un ruban élastique ou de préférence par un ruban de matériau sous forme de mousse élastique, par exemple de mousse de polyuréthane, ou par tout autre matériau élastique sous forme allongée.

## Revendications

1. Article d'hygiène absorbant jetable, notamment couche-culotte, ayant une forme générale sensiblement rectangulaire avec des bords longitudinaux opposés (8, 9) et des bords transversaux opposés (7) et comprenant, de l'extérieur vers l'intérieur, une feuille de support (4) imperméable aux liquides, un coussin absorbant (6) disposé sur la face intérieure de la feuille de support, les dimensions du coussin étant inférieures à celles de la feuille de support, et une feuille de couverture (5) recouvrant la face intérieure du coussin absorbant et de la feuille de support et reliée à la feuille de support sur le pourtour du coussin absorbant, des éléments élastiques longitudinaux (13) étant fixés à l'état tendu à la feuille de support, à l'extérieur des bords longitudinaux (12) du matelas absorbant, et des moyens d'attaches (14) étant prévus au voisinage de l'un des bords transversaux de l'article d'hygiène pour fermer ce dernier autour de la taille d'un utilisateur de telle manière que l'article d'hygiène définisse une partie avant (2) et une partie arrière (1) correspondant respectivement aux deux zones d'extrémité proches desdits bords transversaux opposés de l'article d'hygiène, et une partie d'entre-jambes (3) correspondant à la zone intermédiaire située entre lesdites zones d'extrémité, ledit article comprenant par ailleurs deux volets latéraux (15) espacés transversalement disposés sur la face intérieure de la feuille de couverture, sensiblement le long des bords longitudinaux de l'article d'hygiène lesdits volets présentant chacun une partie proximale (17) reliée à la feuille de couverture et une partie distale (19) comportant des éléments élastiques longitudinaux (20) tendus, et une poche d'étanchéité de ceinture s'étendant, au voisinage d'au moins l'un des bords transversaux de l'article d'hygiène, entre les parties proximales des volets latéraux, en dessous desdits volets, ladite poche étant fixée à l'article d'hygiène par son bord transversal correspondant au bord transversal de l'article d'hygiène ainsi que sur toute ou partie de ses. bords longitudinaux opposés, comprenant, en outre, une feuille supplémentaire (25; 30) perméable aux liquides s'étendant depuis l'un au moins desdits bords transversaux (7) de la feuille de support (4) et de la feuille de couverture (5) au moins par-dessus le bord transversal (10) correspondant du coussin absorbant (6), et que parmi les deux feuilles constituées par la feuille de couverture et ladite feuille supplémentaire, une première (5; 30) qui est la plus éloignée du coussin absorbant, caractérisé par le fait que ladite première feuille (5, 30)
a) comporte, au-dessus du coussin absorbant, au voisinage dudit bord transversal du coussin absorbant, une découpe transversale (23; 32) entre les parties proximales (17) desdits volets latéraux,
b) est munie d'un élément élastique transversal (24; 33) tendu longeant ladite découpe sur le côté tourné vers le bord transversal correspondant de l'article d'hygiène, et
c) est fixée à la seconde (25; 5) desdites deux feuilles, au moins le long dudit bord transversal de l'article d'hygiène et dans le prolongement des deux extrémités dudit élément élastique transversal, pour constituer ainsi une poche d'étanchéité de ceinture ouverte à l'endroit de ladite découpe transversale.

2. Article d'hygiène suivant la revendication 1, caractérisé par le fait que ladite feuille supplémentaire (25) est disposée entre le coussin absorbant (6) et la feuille de couverture (5) et constitue ladite seconde feuille.

3. Article d'hygiène suivant la revendication 1, caractérisé par le fait que ladite feuille supplémentaire (30) perméable aux liquides est disposée sur la face intérieure de ladite feuille de couverture (5) et constitue ladite première feuille.

4. Article d'hygiène suivant l'une quelconque des revendications précédentes, caractérisé par le fait que ladite feuille supplémentaire (25, 30) s'étend d'un bord transversal de l'article d'hygiène à l'autre et que ladite première feuille (5; 30) est fixée à ladite seconde feuille (25; 5) le long des deux bords transversaux (7) de l'article d'hygiène.

5. Article d'hygiène suivant les revendications 2 et 4, caractérisé par le fait que ladite première feuille (5) comporte une découpe transversale (23) et est munie d'un élément élastique transversal tendu (24) au voisinage de chaque bord transversal (10) du coussin absorbant (6) et comporte deux découpes longitudinales (38) joignant entre elles les extrémités desdites deux découpes transversales (23) de manière que lesdites découpes transversales et lesdites découpes longitudinales définissent une ouverture centrale (37) de forme générale rectangulaire dans ladite première feuille (5).

6. Article d'hygiène suivant la revendications 5, caractérisé par le fait que ladite première feuille (5) est hydrophobe ou imperméable aux liquides.

7. Article d'hygiène suivant l'une quelconque des revendications 1 à 4, caractérisé par le fait que la partie de ladite première feuille perméable aux liquides située entre ladite découpe transversale et le bord transversal correspondant de l'article d'hygiène est imperméable aux liquides ou hydrophobe.

8. Article d'hygiène suivant la revendication 7, caractérisé par le fait que ladite partie de ladite première feuille perméable aux liquides comporte un revêtement imperméable ou une enduction ou un autre traitement imperméable ou hydrophobe.

9. Article d'hygiène suivant la revendication 8, caractérisé par le fait que ledit revêtement est réalisé en un matériau élastique et constitue en même temps ledit élément élastique transversal.

10. Article d'hygiène suivant l'une quelconque des revendications précédentes, caractérisé par le fait que ladite fixation de ladite première feuille perméable à ladite seconde feuille perméable s'effectue par un collage en U comprenant une ligne de colle transversale longeant le bord transversal de l'article d'hygiène et deux lignes de colle longitudinales s'étendant le long des bords longitudinaux de ladite première feuille, sur tout ou partie de la longueur desdits bords.

11. Article d'hygiène suivant la revendication 10, caractérisé par le fait que ladite fixation s'effectue par un collage en rectangle avec une ligne transversale supplémentaire longeant ladite découpe du côté éloigné du bord transversal correspondant de l'article d'hygiène.

12. Article d'hygiène suivant l'une quelconque des revendications 3, 4 et 7 à 11, caractérisé par le fait que ladite feuille supplémentaire (30) perméable aux liquides est fixée à la feuille de couverture (5), sur toute la longueur de l'article d'hygiène, par deux lignes de liaison (18) longitudinales en retrait par rapport aux bords longitudinaux (19) de ladite feuille supplémentaire, et que des éléments élastiques (20) sont fixés, à l'état tendu, le long desdits bords longitudinaux de la feuille supplémentaire, les parties (35) de ladite feuille situées entre lesdites lignes longitudinales de liaison et lesdits bords longitudinaux constituant lesdits volets latéraux (15).

13. Article d'hygiène suivant la revendication 12, caractérisé par le fait que que ladite feuille supplémentaire perméable aux liquides est enduite ou traitée imperméable ou hydrophobe dans la zone desdits volets (15).

## Claims

1. Disposable absorbent hygiene article, especially napkin pilch, having a substantially rectangular general shape with opposite longitudinal edges (8, 9) and opposite transverse edges (7) and comprising, from the outside inwards, a supporting sheet (4) impermeable to liquids, an absorbent pad (6) arranged on the internal face of the supporting sheet, the dimensions of the pad being smaller than those of the supporting sheet, and a covering sheet (5) covering the internal face of the absorbent pad and of the supporting sheet and joined to the supporting sheet over the periphery of the absorbent pad, longitudinal elastic elements (13) being fastened in the tensioned state to the supporting sheet on the outside of the longitudinal edges (12) of the absorbent pad, and fastening means (14) being provided in the vicinity of one of the transverse edges of the hygiene article, in order to close the latter around the waist of a user in such a way that the hygiene article defines a front part (2) and a rear part (1) corresponding respectively to the two end zones near said opposite transverse edges of the hygiene article and a crutch part (3) corresponding to the intermediate zone located between said end zones, said article comprising, furthermore, two transversely spaced lateral flaps (15) arranged on the internal face of the covering sheet substantially along the longitudinal edges of the hygiene article, said flaps each having a proximal part (17) joined to the covering sheet and a distal part (19) having tensioned longitudinal elastic elements (20), and a sealing waistband pocket extending, in the vicinity of at least one of the transverse edges of the hygiene article, between the proximal parts of the lateral flaps underneath said flaps, said pocket being fixed to the hygiene article by means of its transverse edge corresponding to the transverse edge of the hygiene article and over all or some of its opposite longitudinal edges , comprising, moreover an additional sheet (25;30) permeable to liquids and extending from at least one of said transverse edges (7) of the supporting sheet (4) and of the covering sheet (5) at least over the corresponding transverse edge (10) of the absorbent pad (6), and in that, of the two sheets formed by the covering sheet and said additional sheet, a first (5; 30), which is furthest from the absorbant pad, characterised in that the said first sheet (5,30)
a) comprises, above the absorbent pad, in the vicinity of said transverse edge of the absorbent pad, a transverse cutout (23; 32) between the proximal parts (17) of said lateral flaps,
b) is equipped with a tensioned transverse elastic element (24; 33) extending along said cutout on the side confronting the corresponding transverse edge of the hygiene article, and
c) is fastened to the second (25; 5) of said two sheets at least along said transverse edge of the hygiene article and in the extension of the two ends of said transverse elastic element, in order thus to form a sealing waistband pocket open at the location of said transverse cutout.

2. Hygiene article according to Claim 1, characterised in that said additional sheet (25) is arranged between the absorbent pad (6) and the covering sheet (5) and forms said second sheet.

3. Hygiene article according to Claim 1, characterised in that said additional sheet (30) permeable to liquids is arranged on the internal face of said covering sheet (5) and forms said first sheet.

4. Hygiene article according to any one of the preceding claims, characterised in that said additional sheet (25, 30) extends from one transverse edge of the hygiene article to the other, and in that said first sheet (5; 30) is fixed to said second sheet (25; 5) along the two transverse edges (7) of the hygiene article.

5. Hygiene article according to Claims 2 and 4, characterised in that said first sheet (5) comprises a transverse cutout (23) and is equipped with a tensioned transverse elastic element (24) in the vicinity of each transverse edge (10) of the absorbent pad (6) and comprises two longitudinal cutouts (38) joining the ends of said two transverse cutouts (23) to one another, in such a way that said transverse cutouts and said longitudinal cutouts define a central orifice (37) of general rectangular shape in said first sheet (5).

6. Hygiene article according to Claim 5, characterised in that said first sheet (5) is hydrophobic or impermeable to liquids.

7. Hygiene article according to any one of Claims 1 to 4, characterised in that the part of said first liquid-permeable sheet located between said transverse cutout and the corresponding transverse edge of the hygiene article is impermeable to liquids or hydrophobic.

8. Hygiene article according to Claim 7, characterised in that said part of said first liquid-permeable sheet comprises an impermeable covering or impermeable or hydrophobic coating or other treatment.

9. Hygiene article according to Claim 8, characterised in that said covering is produced from an elastic material and forms at the same time said transverse elastic element.

10. Hygiene article according to any one of the preceding claims, characterised in that said fixing of said first permeable sheet to said second permeable sheet takes place by means of a U-shaped adhesive bond comprising a transverse line of adhesive extending along the transverse edge of the hygiene article and two longitudinal lines of adhesive extending along the longitudinal edges of said first sheet over all or some of the length of said edges.

11. Hygiene article according to Claim 10, characterised in that said fixing takes place by means of a rectangular adhesive bond with an additional transverse line extending along said cutout on the side remote from the corresponding transverse edge of the hygiene article.

12. Hygiene article according to any one of Claims 3, 4 and 7 to 11, characterised in that said additional sheet (30) permeable to liquids is fixed to the covering sheet (5) over the entire length of the hygiene article by means of two longitudinal connecting lines (18) set back relative to the longitudinal edges (19) of said additional sheet, and in that elastic elements (20) are fixed in the tensioned state along said longitudinal edges of the additional sheet, the parts (35) of said sheet which are located between said longitudinal connecting lines and said longitudinal edges forming said lateral flaps (15).

13. Hygiene article according to Claim 12, characterised in that said additional sheet permeable to liquids is coated or treated so as to be impermeable or hydrophobic in the region of said flaps (15).

## Patentansprüche

1. Wegwerfbarer, absorbierender Hygieneartikel, insbesondere Höschenwindel, die eine allgemein im wesentlichen rechteckige Form mit einander gegenüberliegenden longitudinalen Rändern (8, 9) und gegenüberliegenden, transversalen Rändern (7) aufweist, und die, von außen nach innen hin, umfaßt: eine Tragfolie (9), die für Flüssigkeiten undurchlässig ist, ein absorbierendes Kissen (6), das auf der inneren Seite der Tragfolie angeordnet ist, wobei die Abmessungen des Kissens kleiner als diejenigen der Tragfolie sind, und eine Abdeckfolie (5), die die innere Seite des absorbierenden Kissens und der Tragfolie überdeckt, und die mit der Tragfolie entlang der Peripherie des absorbierenden Kissens verbunden ist, longitudinale, elastische Elemente (13), die im gespannten Zustand an der Tragfolie außerhalb der longitudinalen Ränder (12) des absorbierenden Polsterkissens befestigt sind, und Befestigungsmittel (14), die in der Nähe des einen der transversalen Ränder des Hygieneartikels vorgesehen sind, um letzteren um die Taille eines Benutzers herum zu befestigen, derart, daß der Hygieneartikel einen vorderen Abschnitt (2) und einen hinteren Abschnitt (1) entsprechend jeweils den beiden proximalen Endzonen der einander gegenüberliegenden transversalen Ränder des Hygieneartikels, sowie einen Oberschenkelzwischenabschnitt (3) entsprechend der Zwischenzone definieren, die sich zwischen den genannten Endzonen befindet; wobei der Artikel darüber hinaus zwei transversal beabstandete, seitliche Flügel (15) umfaßt, die auf der inneren Seite der Abdeckfolie im wesentlichen entlang der longitudinalen Ränder des Hygieneartikel angeordnet sind; wobei die genannten Flügel jeweils einen proximalen Abschnitt (17), welcher mit der Abdeckfolie verbunden ist, und einen distalen Abstand (19) aufweisen, der gespannte, longitudinale, elastische Elemente (20) umfaßt; und wobei der Artikel eine Gürtelabdichttasche aufweist, die sich in der Nähe mindestens des einen der transversalen Ränder des Hygieneartikels zwischen den proximalen Abschnitten der seitlichen Flügel und unter den genannten Flügeln erstreckt; wobei die genannte Tasche am Hygieneartikel durch ihren entsprechenden transversalen Rand am transversalen Rand des Hygieneartikels sowie über die gesamte Länge oder einen Teil derselben ihrer einander gegenüberliegenden, longitudinalen Ränder hinweg befestigt ist; und daß der Hygieneartikel weiter eine zusätzliche Folie (25; 30) umfaßt, die für Flüssigkeiten durchlässig ist und sich, beginnend bei dem mindestens einen der transversalen Ränder (7) der Tragfolie (4) und der Abdeckfolie (5) mindestens über den entsprechenden transversalen Rand (10) des absorbierenden Kissens (6) hinweg erstreckt; und daß von den beiden Folien, bestehend aus der Abdeckfolie und der genannten zusätzlichen Folie, eine erste Folie (5; 30), welche die vom absorbierenden Kissen am weitesten entfernte ist,
dadurch **gekennzeichnet** ist, daß die genannte erste Folie (5; 30):
a) über dem absorbierenden Kissen in der Nähe des genannten transversalen Randes des absorbierenden Kissens einen transversalen Einschnitt (23; 32) zwischen den proximalen Abschnitten (17) der genannten seitlichen Flügel aufweist,
b) mit einem gespannten, transversalen, elastischen Element (24; 33) versehen ist, das entlang der Seite des Einschnittes verläuft, welche zum entsprechenden transversalen Rand des Hygieneartikels hingewendet ist, und
c) an der zweiten Folie (25; 5) der beiden Folien mindestens entlang des genannten transversalen Randes des Hygieneartikels sowie in der Verlängerung der beiden Enden des genannten transversalen elastischen Elementes befestigt ist, um so eine offene Gürtelabdichttasche an der Stelle des transversalen Einschnittes zu bilden.

2. Hygieneartikel nach Anspruch 1,
dadurch **gekennzeichnet,** daß die zusätzliche Folie (25) zwischen dem absorbierenden Kissen (6) und der Abdeckfolie (5) angeordnet ist und die genannte zweite Folie bildet.

3. Hygieneartikel nach Anspruch 1,
dadurch **gekennzeichnet,** daß die zusätzliche Folie (30), die für Flüssigkeiten durchlässig ist, auf der inneren Seite der genannten Abdeckfolie (5) angeordnet ist und die genannte erste Folie bildet.

4. Hygieneartikel nach einem beliebigen vorhergehenden Anspruch,
dadurch **gekennzeichnet,** daß die zusätzliche Folie (25, 30) sich von einem transversalen Rand des Hygieneartikels zum anderen hin erstreckt, und daß die genannte erste Folie (5; 30) an der zweiten Folie (25; 5) entlang der beiden transversalen Ränder (7) des Hygieneartikels befestigt ist.

5. Hygieneartikel nach den Ansprüchen 2 und 4,
dadurch **gekennzeichnet,** daß die genannte erste Folie (5) einen transversalen Einschnitt (23) aufweist und mit einem gespannten, transversalen, elastischen Element (24) in der Nähe jedes transversalen Randes (10) des absorbierenden Kissens (6) versehen ist und zwei longitudinale Einschnitte (38) aufweist, die sich untereinander mit den genannten Enden der beiden transversalen Einschnitte (23) verbinden, derart, daß die transversalen Einschnitte und die longitudinalen Einschnitte eine zentrale Öffnung (37) von allgemein rechteckiger Form in der ersten Folie (5) definieren.

6. Hygieneartikel nach Anspruch 5,
dadurch **gekennzeichnet,** daß die erste Folie (5) wasserabweisend oder für Flüssigkeiten undurchlässig ist.

7. Hygieneartikel nach einem beliebigen Anspruch 1 bis 4,
dadurch **gekennzeichnet,** daß der Abschnitt der für Flüssigkeiten durchlässigen ersten Folie, der sich zwischen dem genannten transversalen Einschnitt und dem entsprechenden transversalen Rand des Hygieneartikels befindet, für Flüssigkeiten undurchlässig oder wasserabweisend ist.

8. Hygieneartikel nach Anspruch 7,
dadurch **gekennzeichnet,** daß der genannte Abschnitt der für Flüssigkeiten durchlässigen ersten Folie einen undurchlässigen Überzug oder eine Beschichtung oder eine andere Bearbeitung aufweist, die undurchlässig oder wasserabweisend wirkt.

9. Hygieneartikel nach Anspruch 8,
dadurch **gekennzeichnet,** daß der genannte Überzug aus einem elastischen Material gebildet ist und gleichzeitig das transversale, elastische Element bildet.

10. Hygieneartikel nach einem beliebigen vorherigen Anspruch,
dadurch **gekennzeichnet,** daß die Befestigung der genannten durchlässigen ersten Folie an der durchlässigen zweiten Folie durch eine U-förmige Klebung erfolgt, die eine transversale Kleberlinie umfaßt, welche neben dem transversalen Rand des Hygieneartikels verläuft, und die zwei longitudinale Kleberlinien umfaßt, die sich entlang der longitudinalen Ränder der genannten ersten Folie über die gesamte Länge oder über einen Teil der Länge der genannten Ränder erstrecken.

11. Hygieneartikel nach Anspruch 10,
dadurch **gekennzeichnet,** daß die Befestigung durch eine rechteckförmige Klebung mit einer zusätzlichen, transversalen Linie erfolgt, welche entlang des genannten Einschnittes auf der vom entsprechenden transversalen Rand des Hygieneartikels entfernt liegenden Seite verläuft.

12. Hygieneartikel nach einem beliebigen Anspruch 3, 4 und 7 bis 11,
dadurch **gekennzeichnet,** daß die für Flüssigkeiten durchlässige, zusätzliche Folie (30) an der Abdeckfolie (5) über die gesamte Länge des Hygieneartikels durch zwei longitudinale Verbindungslinien (18) befestigt ist, die gegen die longitudinalen Ränder (19) der genannten zusätzlichen Folie rückgesetzt sind, und daß die elastischen Elemente (20) im gespannten Zustand entlang der genannten longitudinalen Ränder der zusätzlichen Folie befestigt sind, wobei die Abschnitte (35) der genannten Folie, welche sich zwischen den genannten longitudinalen Verbindungslinien und den genannten longitudinalen Rändern befinden, die genannten seitlichen Flügel (15) bilden.

13. Hygieneartikel nach Anspruch 12,
dadurch **gekennzeichnet,** daß die für Flüssigkeiten durchlässige, zusätzliche Folie in der Zone der genannten Flügel (15) beschichtet oder undurchlässig gemacht, oder wasserabweisend ist.
